# EUROPEAN PATENT SPECIFICATION

(11) **EP 3 166 563 B1**
(45) Date of publication and mention of the grant of the patent: **16.12.2020**
(21) Application number: 15733618.1
(22) Date of filing: 30.06.2015
(51) Int. Cl.: A61J 1/00, A61J 7/00, A61M 11/00, A61K 9/70, A61K 31/465, A61K 47/36, A61M 15/00

(54) **ORAL DELIVERY SYSTEM COMPRISING TWO COMPARTMENTS**
ORALES ABGABESYSTEM MIT ZWEI FÄCHERN
SYSTÈME D'ADMINISTRATION ORALE COMPRENANT DEUX COMPARTIMENTS

(30) Priority: 08.07.2014 DK 201470424
(43) Date of publication of application: 17.05.2017
(73) Proprietor: Fertin Pharma A/S, 7100 Vejle (DK)
(72) Inventor: NIELSEN, Bruno Provstgaard, DK-7120 Vejle Øst (DK)
(74) Representative: Patentgruppen A/S
(86) International application number: PCT/DK2015/050192
(87) International publication number: WO 2016/004952

(56) References cited:
- WO-A1-98/47535
- WO-A1-2013/143891
- WO-A2-2006/100075
- US-A- 5 147 648

## Description

### Field of invention

The invention relates to an oral delivery system according to claim 1.

### Background

Several oral delivery systems are known within the art. Several problems exist in connection with such oral delivery systems. For example, when using these oral delivery systems to delivery active ingredients to the oral cavity, the delivery may be inaccurate or the active ingredient may not reach its intended target, such as the oral mucosa.

### Summary of the invention

An oral delivery system comprising a first compartment and a second compartment,
said first compartment comprising a first component comprising natural unbranched polysaccharide, said second compartment comprising a second component comprising multivalent cations,
wherein the oral delivery system is adapted for administering the first and second components in a synchronized manner to the oral cavity,
whereby a bioadhesive gel is formed from said natural unbranched polysaccharide and said multivalent cations in a cross-linking reaction.

One important feature of the present invention may be that the oral delivery system and/or its content and components is/are adapted for oral use, e.g. to prevent poisoning, which may often occur if applying preparations intended for use elsewhere.

It should be stressed that oral use may pose unique limitations to the delivery system and/or its contents, e.g. due to increased absorption of content or active ingredients therein. Therefore, when using other delivery systems not intended for oral use as an oral delivery this may lead to poisoning or even a life-threatening state for the user.

One further important advantage of the present invention may be that it may be relatively easy and simple to use. Due to the fact that the present oral delivery system may often be used by ordinary private home users, the simplicity as well as a relatively low production price may be an exceptionally important advantage.

One further advantage of the invention may be that use of taste masking agents may be reduced or even avoided, since the components may be adjusted, under the circumstances, to reduce or even avoid unpleasant taste.

According to an advantageous embodiment of the invention, said cross-linking reaction occurs at least partly in the oral cavity.

One advantage of the present invention may be that a reusable product may be obtained. Any pre-mixing prior to administration may be avoided. This may be important, for example, when the first and second components are in the liquid state, according to these embodiments, no gelation occurs in the delivery system, avoiding any clogging at common outlet openings. Typically, such common outlet openings would often lead to clogging, e.g. due to the fact that after the desired amount of components have been administered, some residue mixture may typically be left in the mixing device or output opening itself. After a short time, this residue would form a gel obstructing subsequent administration of the components. However, by means of the solution of the invention, the oral cavity is utilized as a mixing reactor thereby avoiding clogging and giving a reusable delivery system. Since the use of the oral delivery system may often be by ordinary unskilled persons, it is important that the device can be manufactured and sold for a relatively affordable price and that it is relatively easy to use.

Also, one further advantage of avoiding pre-mixing may be that premature gel formation may be avoided or at least reduced. If the gel forms too early, it may prevent or obstruct migration in the oral cavity to the target mucosa, such as the oral mucosa. Therefore, by using the oral cavity as a mixing reactor the gel may be formed at or at least relatively close to the target mucosa thereby increasing the amount of bio-adhesive gel actually adhered to the mucosa.

According to an embodiment of the invention, at least 40 % of the said cross-linking reaction occurs in the oral cavity, such as at least 50%, such as at least 60%, such as at least 70%, such as at least 80%, such as at least 90%, such as at least 95%.

It may in fact be an advantage of the above embodiment that a relatively large fraction of said cross-linking reaction occurs in the oral cavity, e.g. due to the fact that the viscosity of a mixture of said first and second components may increase drastically during said cross-linking reaction and that the pre-cross-linking reaction mixture therefore may be more mobile and more easily cover the entire mucosa before gelling.

According to an advantageous embodiment of the invention, the oral delivery system is an oral spray system.

An oral delivery system in the form of a spray system may in many cases be advantageous. One advantage may e.g. be when said administered substances, including said first and second components, are found as liquid suspensions or solutions. Administering such solutions or suspensions, which would have a viscosity lower the final bioadhesive gel, to the oral cavity, may allow some flow of the first and/or second ingredient before the cross-linking reaction has facilitated in full the increase in viscosity of the mixture by formation of the bioadhesive gel.

According to an advantageous embodiment of the invention, the first and/or second components comprise(s) at least one active ingredient.

The oral delivery system may advantageously be utilized as a vehicle for delivering one or more active ingredients to the oral cavity. For example, by including a pharmaceutical compound as an active ingredient in the first and/or second component, the oral delivery system may be utilized as a delivery vehicle for delivering the pharmaceutical compound to the oral cavity, where the pharmaceutical compound may be absorbed into the body, e.g. through the oral mucosa.

According to an advantageous embodiment of the invention, said active ingredient comprises nicotine.

One significant advantage of the above embodiment may be that delivery of nicotine to the oral cavity is facilitated, from where it may be absorbed through the oral mucosa. Therefore, an oral delivery system according to the above embodiment may deliver nicotine to the oral cavity, where it may be immobilized in the bioadhesive gel. Thereby, it is avoided that too much of the nicotine, which may be rather expensive, is swallowed or otherwise escapes the oral cavity without being absorbed through the oral mucosa.

According to an advantageous embodiment of the invention, said nicotine is selected from the group consisting of a nicotine salt, the free base form of nicotine, a nicotine derivative, such as a nicotine cation exchanger, such as nicotine polacrilex resin, a nicotine inclusion complex or nicotine in any non-covalent binding; nicotine bound to zeolites; nicotine bound to cellulose, such as microcrystalline, or starch microspheres, and mixtures thereof.

According to an advantageous embodiment of the invention, the nicotine salt is selected from the group comprising nicotine hydrochloride, nicotine dihydrochloride, nicotine monotartrate, nicotine bitartrate, nicotine sulfate, nicotine zinc chloride, nicotine salicylate, or any combination thereof.

According to an advantageous embodiment of the invention, nicotine is present as a nicotine polacrilex resin.

According to an advantageous embodiment of the invention, nicotine is added to the first component and/or the second component as tobacco powder.

According to an advantageous embodiment of the invention, at least a part of the nicotine is contained in tobacco powder.

In an embodiment of the invention, the nicotine is selected from the group consisting of a nicotine salt, the free base form of nicotine, a nicotine derivative, such as a nicotine cation exchanger, such as nicotine polacrilex resin, a nicotine inclusion complex or nicotine in any non-covalent binding; nicotine bound to zeolites; nicotine bound to cellulose, such as microcrystalline, or starch microspheres, and mixtures thereof.

It is generally noted that specific examples and explanation specifically referring to nicotine as an active pharmaceutical ingredient against craving in no way restricts the scope of the invention with respect to use of other tobacco alkaloids for the same specific purpose. A specific mentioning of nicotine at any place in this application is only used for the purpose of exemplifying the invention in a tangible way and not for the purpose of excluding alternative functionally equivalents.
It should generally be noted that nicotine is a preferred tobacco alkaloid.

In the present invention, the term nicotine encompasses nicotine or a nicotine derivative in any form such as, e.g. physical forms like amorphous, crystalline, polymerphous etc. or chemical form like isomers and enantiomers etc. as well as any pharmaceutically acceptable salts, complex or solvate thereof. Nicotine may be selected from nicotine base, nicotine hydrochloride, nicotine dihydrochloride, nicotine monotartrate, nicotine bitartrate, nicotine sulfate, nicotine zinc chloride such as zinc chloride monohydrate and nicotine salicylate.

In an embodiment of the invention, the nicotine salts are selected from the group comprising nicotine hydrochloride, nicotine dihydrochloride, nicotine monotartrate, nicotine bitartrate, nicotine sulfate, nicotine zinc chloride, nicotine salicylate, or any combination thereof.

According to an advantageous embodiment of the invention, said first component and second component are adapted to form a bio-adhesive gel which adheres at least partly to the oral mucosa.

According to an advantageous embodiment of the invention, said first component and second component are adapted for forming a bioadhesive gel adhering to the oral mucosa in an amount of at least 20 % by weight of the formed bioadhesive gel, such as at least 30 % by weight, such as at least 40 % by weight, such as at least 50 % by weight, such as at least 60 % by weight, such as at least 70 % by weight, such as at least 80 % by weight, such as at least 90 % by weight.

By the above by is illustrated the amount of the formed bioadhesive gel adhering to the oral mucosa, i.e. the part of the formed bioadhesive gel being part of e.g. a bioadhesive film formed on the oral mucosa.

According to an advantageous embodiment of the invention, said first component and/or second component comprise(s) a pH-controlling agent.

By including a pH-controlling agent in the first and/or second component several advantages may be obtained. One advantage of the above embodiment may be that by affecting the pH-level in the mouth to a desired pH-level, increased absorption of nicotine through the oral mucosa may be facilitated.

Alternatively, the oral delivery system may comprise a third compartment including a pH-controlling agent, which is also administered synchronized, e.g. substantially at the same time, as the first and second components. The third compartment may comprise nicotine.

According to an advantageous embodiment of the invention, said pH-controlling agent comprises a buffer.

In an embodiment of the invention, the first and/or second component comprises buffer in the amount of ½ to 5% by weight of the first and/or second component, such as 1 to 4 %, such as 2 to 5 %, such as 3 to 5 %, such as 3 to 4 %, such as 1 to 3 %.

In an embodiment of the invention, the buffer is selected from the group consisting of a carbonate, including bicarbonate or sesquicarbonate, glycerinate, phosphate, glycerophosphate, acetate, glyconate or citrate of an alkali metal, such as potassium or sodium, e.g. trisodium and tripotassium citrate, or ammonium, tris buffer, amino acids, and mixtures thereof.

In an embodiment of the invention, the buffer comprises sodium carbonate, sodium bicarbonate or any combination thereof.

In an embodiment of the invention, the buffer comprises sodium carbonate, sodium bicarbonate or potassium carbonate.

According to a preferred embodiment of the invention, the buffer comprises sodium carbonate.

In connection to the above, it should be understood that in embodiments where said oral delivery system is a spray, said buffer may advantageously be in the form of a liquid solution or suspension so as to facilitate the administration of said buffer. Furthermore, in embodiments where said oral delivery system is in the form of a chewing gum or a lozenge, said buffer may advantageously be in the form of a solid, such as e.g. a powder.

According to an advantageous embodiment of the invention, said pH-controlling agent comprises an acid.

According to an advantageous embodiment of the invention, said oral delivery system is adapted for delivering a pre-defined amount of said active ingredient to the oral cavity.

One advantage of the above embodiment may be that the end user using the oral delivery system can administer a predefined amount of said active ingredient to him- or herself, and thereby also get an expected effect corresponding to the predefined amount. One further advantage may be that while achieving the above advantage, it is at the same time secured that not too much of the active ingredient is administered. Especially in embodiments, where the active ingredient is e.g. nicotine, this may be very important, as a too high dosage of such compounds may give rise to serious side effects or even life threatening situations.

According to an advantageous embodiment of the invention, said pre-defined amount is 0.1 - 4 mg, such as 0.1 - 1 mg, or such as 1 - 2 mg, or such as 2 - 3 mg, or such as 3 - 4 mg.

The above interval may be especially preferred when the active ingredient is nicotine.

According to an example embodiment, said oral delivery system is adapted for delivering a pre-defined amount of nicotine to the oral cavity, where said pre-defined amount is 0.1 - 3 mg, such as 0.1 - 2 mg, such as 0.1 - 1 mg, such as 0.2 - 1 mg, such as 0.5 - 1 mg.

The above interval may be especially preferred when the active ingredient is nicotine.

According to an example embodiment, said oral delivery system is adapted for delivering a pre-defined amount of nicotine to the oral cavity, where said pre-defined amount is 0.5 - 3 mg, such as 0.5 - 2 mg, such as 0.5 - 1 mg.

The above interval may be especially preferred when the active ingredient is nicotine.

According to an example embodiment, said oral delivery system is adapted for delivering a pre-defined amount of nicotine to the oral cavity, where said pre-defined amount is 0.5 - 4 mg, such as 1 - 3 mg, such as 2 - 3 mg.

The above interval may be especially preferred when the active ingredient is nicotine.

According to an example embodiment, said oral delivery system is adapted for delivering a pre-defined amount of nicotine to the oral cavity, where said pre-defined amount is 0.5 - 4 mg, such as 1 - 4 mg, such as 2 - 4 mg, such as 3 - 4 mg.

The above interval may be especially preferred when the active ingredient is nicotine.

According to an advantageous embodiment of the invention, said pre-defined amount between a series of at least 10 of administrations from said oral delivery system holds a relative standard deviation of at most 6%.

According to an advantageous embodiment of the invention, said first component is in the form of aqueous solution.

According to the above embodiment, it may be advantageous to deliver the first component, possibly comprising active ingredients, to the oral cavity in as an aqueous solution to allow the first component to flow or diffuse before the bioadhesive gel is formed, thereby possibly obtaining a greater contact interface area between the bioadhesive gel and the oral mucosa. This may be particularly advantageously when the first and/or second component comprises an active ingredient, such as nicotine, which may then be effectively positioned over a larger portion of the surface of the oral mucosa, thereby facilitating effective and/or long-lasting absorption of the active ingredient over the mucous membrane of the oral mucosa and into the body.

According to an advantageous embodiment of the invention, said first component is in the form of an aqueous suspension.

According to the above embodiment, it may be advantageous to deliver the first component, possibly comprising active ingredients, to the oral cavity in as an aqueous suspension to allow the first component to flow or diffuse before the bioadhesive gel is formed, thereby possibly obtaining a greater contact interface area between the bioadhesive gel and the oral mucosa. This may be particularly advantageously when the first and/or second component comprises an active ingredient, such as nicotine, which may then effectively positioned over a larger portion of the surface of the oral mucosa, thereby facilitating effective and long-lasting absorption of the active ingredient over the mucous membrane of the oral mucosa and into the body.

According to an advantageous embodiment of the invention, said first component is in the form of a solid.

It may be advantageous to provide said first component as a solid, such as a powder, thereby avoiding liquid suspensions and solutions, which may in some cases be preferred. For example, by providing the first component as a solid, e.g. in the form of a chewing gum or a lozenge, an oral delivery system is obtained, which is relatively simple for the end user to administrate to the oral cavity in correct amounts.

According to an advantageous embodiment of the invention, said first component is in the form of a powder.

According to an advantageous embodiment of the invention, said natural unbranched polysaccharide is in the form of a powder.

According to an advantageous embodiment of the invention, said first component is encapsulated.

According to an advantageous embodiment of the invention, said al unbranched polysaccharide is encapsulated.

According to an advantageous embodiment of the invention, said natural unbranched polysaccharides comprise alginate.

One advantage of the above embodiment may be that alginate is that it is relatively cheap. One further advantage of the above embodiment may be that alginate is relatively tasteless, thereby being suitable for being administered to the oral cavity without too much taste masking agent, such as flavors and/or sweeteners, and without or least only with a minimum of taste displeasure for the person to whom the alginate is administered. One further advantage of the above embodiment may be that alginate is non-toxic and therefore administration of alginate to the oral cavity is safe. One further advantage of the above embodiment may be that a bioadhesive gel may be formed from alginate in a cross-linking reaction, with a suitable second component.

One further significant advantage is that a relatively effective bioadhesive gel may be formed that can facilitate maintaining an active ingredient, such as nicotine, on or in the immediate vicinity of the oral cavity, thereby resulting in an effective absorption of nicotine.

According to an advantageous embodiment of the invention, said natural unbranched polysaccharides is alginate.

According to an advantageous embodiment of the invention, said alginate is in the form of alginic acid, alkaline earth metal alginate, such as sodium alginate or potassium alginate, strontium alginate, polyethylene glycol alginate, NHU-alginate, or any combinations thereof.

According to an advantageous embodiment of the invention, said first component has a viscosity in the range of 1-10 cP (centipoise), such as 5 cP, or in the range of 10-100 cP, such as 50 cP, or in the range of 100 -800 cP, such as 400 cP, or in the range of 800-2000 cP, such as 1400 cP.

According to an advantageous embodiment of the invention, said natural unbranched polysaccharide is present in said first component in a concentration of 0.1 to 15 % by weight, such as 0.5-10% by weight, such as 1-8% by weight, such as 1.5-5% by weight or 2-7% by weight or 1-3% by weight.

According to an advantageous embodiment of the invention, said multivalent cations comprise metal cations.

One advantage of the above embodiment may be that an effective and non-toxic bioadhesive gel may be formed from the first and second components, which may adhere to and protect the oral cavity and/or facilitate delivery of an active ingredient, such as nicotine, through the oral mucosa and into the body.

According to an advantageous embodiment of the invention, said multivalent cations are selected from the group of divalent cations.

According to an advantageous embodiment of the invention, said multivalent cations are selected from the group consisting of multivalent ions of calcium, magnesium, zinc, barium, iron, manganese, copper, lead, cobalt, nickel, such as Ca²⁺, Mg²⁺, Zn²⁺, Ba²⁺, Fe²⁺, Fe³⁺, Fe⁴⁺, Mn²⁺, Mn⁴⁺, Cu⁴⁺, or combinations thereof.

In a preferred embodiment of the invention, the multivalent cation is selected from the group of Ca²⁺ and Mg²⁺.

In a preferred embodiment of the invention, the multivalent cation is Ca²⁺. This may be preferred in part due non-toxicity of calcium ions, in part due to tastelessness of calcium ions, and in part due to an effective formation of bioadhesive gel.

According to an advantageous embodiment of the invention, said multivalent cations are in the form of a salt, e.g. with organic anions like citrate, lactate, aspartate, saccharate, oxovalerate, gluconate, lactonionate, lactogluconate, or with carbonate or chloride ions.

According to an advantageous embodiment of the invention, said multivalent cations are in the form of aqueous solution.

I.e. according to the above embodiment said second component comprises an aqueous solution comprising said multivalent cations.

According to an advantageous embodiment of the invention, said multivalent cations are in the form of aqueous solution with a pH-level within the range of 1-8, such as 2-7, such as 3-6.5, such as 3-6, such as 4-6.

By controlling the pH-level of said second component, being an aqueous solution of multivalent cations, the solubility of said multivalent cations may be controlled. It may, as an example, when using a second component comprising Ca²⁺ ions, be important to secure that these ions are in the solution and not as precipitates. Thereby high mobility of the Ca²⁺ ions may be facilitated, allowing the Ca²⁺ ions to effectively mix with said natural unbranched polysaccharides and trigger the cross-linking reaction.

In embodiments where the oral delivery system is adapted to administrate nicotine in a synchronized manner with said first and second component, it may be advantageous to administrate the nicotine separately with a pH controlling agent, such as a buffer. For example, the nicotine and the buffer may be administrated just before the first and second components, whereby the first and second component may form a bioadhesive film over the nicotine, thereby maintaining the nicotine on the oral mucosa, and where the pH-controlling agent administered with the nicotine is at the same time keeps the pH-level in the mouth, locally where the nicotine is, at a level facilitating nicotine absorption, such as e.g. at pH levels of 7-9, such as 8-8.5.

According to an advantageous embodiment of the invention, said multivalent cations are comprised in a salt, or in an aqueous suspension or solution of said salt.

I.e. according to the above embodiment said second component comprises an aqueous solution or suspension comprising a salt, said salt comprising said multivalent cations.

According to an advantageous embodiment of the invention, said second component is in the form of a solid.

I.e. according to the above embodiment, said second component is in the form of a solid, said solid comprising said multivalent cations.

According to an advantageous embodiment of the invention, said second component is in the form of a powder.

I.e. according to the above embodiment, said second component is in the form of a powder, said powder comprising said multivalent cations.

According to an embodiment of the invention said powder comprising said multivalent cations have an average powder particle size of e.g. less than 25 µm, 25-45 µm, 45 - 75 µm, 75-125 µm, above 125 µm.

According to an embodiment of the invention at least 50% by weight of said powder comprising said multivalent cations are made up of particles, at least a portion of the particles having a particle size of e.g. less than 25 µm, 25-45 µm, 45 - 75 µm, 75-125 µm, above 125 µm,
wherein said portion may be at least 60% by weight, such as at least 70% by weight, such as at least 80% by weight, such as at least 90% by weight.

According to an embodiment of the invention said powder comprising said multivalent cations are a nanoparticulate.

In should be understood in connection to the above embodiments that in some case it may be possible to increase the strength of said bioadhesive gel by increasing the particle size of said powder comprising said multivalent cations.

According to an advantageous embodiment of the invention, said multivalent cations are encapsulated.

According to an advantageous embodiment of the invention, said multivalent cations are present in said second component in a concentration of 0.0005 to 1 M, such as 0.001 to 0.5 M, such as 0.002 to 0.4 M, such as 0.005 to 0.3 M, such as 0.001 o 0.2 M, such as 0.01 o 0.1 M.

According to an advantageous embodiment of the invention, said first component and/or second component comprise(s) a gelling time controlling substance.

According to an advantageous embodiment of the invention, said gelling time controlling substance comprises a gelling delaying substance.

According to an advantageous embodiment of the invention, said gelling delaying substance comprises a polyol, such as sorbitol.

According to an advantageous embodiment of the invention, said gelling time controlling substance comprises a gelling accelerating substance.

According to an advantageous embodiment of the invention, said gelling accelerating substance comprises a pH-controlling substance, such as a buffer and/or an acid.

In advantage of the above embodiment may be that controlling the pH-level may be important to enhance formation of the bioadhesive gel, e.g. by facilitating proper solubility of said multivalent cations in an aqueous solution.

According to an advantageous embodiment of the invention, said oral delivery system is adapted for administration of said first component and second component substantially at the same time.

According to an advantageous embodiment of the invention, said oral delivery system is adapted for administration of said first component and second component at the same time.

According to an advantageous embodiment of the invention, said oral delivery system is adapted for administration of said first component and second component with at least partly temporal overlap of said first component and said second component.

According to an advantageous embodiment of the invention, said oral delivery system is adapted for administration of said first component and second component with a time difference of less than 5 minutes, such as less than 3 minutes, such as less than 2 minutes, such as less than 1 minute, such as less than 45 seconds, such as less than 30 seconds, such as less than 20 seconds, such as less than 15 seconds, such as less 10 seconds, such as less than 5 seconds, such as less than 2 seconds, such as less than 1 second, such as less than 0.5 second, such as less than 0.2 seconds, such as less than 0.1 second, such as substantially simultaneous.

According to an advantageous embodiment of the invention, said oral delivery system is adapted for administration of said first component and second component with a predefined time difference of less than 5 minutes, such as less than 3 minutes, such as less than 2 minutes, such as less than 1 minute, such as less than 45 seconds, such as less than 30 seconds, such as less than 20 seconds, such as less than 15 seconds, such as less 10 seconds, such as less than 5 seconds, such as less than 2 seconds, such as less than 1 second, such as less than 0.5 second, such as less than 0.2 seconds, such as less than 0.1 second, such as substantially simultaneous.

According to an advantageous embodiment of the invention, said oral delivery system is adapted for administrating said first and second components in such a way that the administrating is terminated with an administration solely of said first or said second component, thereby minimizing or avoiding any residue mixture of said first and second component.

One important advantage of the above embodiment, especially for oral delivery systems, such as spray systems, comprising a common output opening for administering both said first and second components, may be that by avoiding or minimizing residue mixture of said first and second components in the common output opening. Such residue mixture may in fact gel in the common output opening, thereby leading to fully or partial clogging of the common output opening. However, with oral delivery system of the above embodiment, such clogging may be reduced or even avoided, facilitating a reusable oral delivery system, which can make several subsequent administrations to the oral cavity of said first and second components.

It may be advantageous to terminate with only with a component other than the first component, such as said second component.

According to an advantageous embodiment of the invention, said oral delivery system is chosen from the list consisting of a spray, such as a mouth spray, a chewing gum, such as a multi-module chewing gum, a lozenge, such as a multi-module lozenge, a mouth wash container system, such as a compartmentalized mouth wash container system, a drink container system, such as a compartmentalized drink container system, a tablet, such as a multi-module tablet.

According to an advantageous embodiment of the invention, said oral delivery system comprises a third compartment, said third compartment comprises a third component.

According to an advantageous embodiment of the invention, said third component comprises a solvent.

According to an advantageous embodiment of the invention, said third component comprises an active ingredient, such as nicotine.

According to an advantageous embodiment of the invention, said first component is adapted to have a viscosity sufficient to facilitate at least some adhesion to the skin of oral mucosa, preferably even before gelling.

According to an embodiment of the invention, the viscosity sufficient to facilitate at least some adhesion to the skin of oral mucosa, preferably even before gelling, is facilitated by said natural unbranched polysaccharide.

According to an advantageous embodiment of the invention, said natural unbranched polysaccharide comprise one or more functional groups facilitating bioadhesion.

According to an advantageous embodiment of the invention, said bioadhesive gel is effective to cover the oral mucosa, so as to protect from gastric acid, enzymes, bacteria, or other harmful influences.

According to an advantageous embodiment of the invention, said bioadhesive gel is biodegradable.

According to an advantageous embodiment of the invention, said administration triggers in situ formation of said bioadhesive gel.

According to an advantageous embodiment of the invention, said oral delivery system is adapted to administrate said first component in a first amount and said second component in second amount, where said first and second amounts are adapted to each other, e.g. to give a ratio between the natural unbranched polysaccharide and the multivalent ions of between 1:100 and 100:1, such as between 1:50 and 50:1, such as between 1:25 and 25:1, such as between 1:10 and 10:1, such as between 1:5 and 5:1, such as between 1:3 and 3:1, such as between 1:2 and 2:1, such as between 1:1.5 and 1.5:1.

According to an advantageous embodiment of the invention, said oral delivery system is encased in a oral delivery system housing.

According to an advantageous embodiment of the invention, said oral delivery system comprises a battery inside said housing for powering said oral delivery system (ODS).

According to an advantageous embodiment of the invention, said oral delivery system is the form of a portable unit.

According to an advantageous embodiment of the invention, said components are mixed prior to being administered through a common output opening.

According to an advantageous embodiment of the invention, said components are mixed after being administered through a common output opening.

According to an embodiment of the invention, said components are mixed after partly before and partly after being administered through a common output opening.

According to an advantageous embodiment of the invention, said oral delivery system comprises a cartridge, said cartridge comprising at least said first and second compartments and optionally further compartments, said cartridge being replaceable.

According to an advantageous embodiment of the invention, said oral delivery system comprises a limiter arrangement for setting a predefined upper limit of the amount of nicotine administrated.

According to an advantageous embodiment of the invention, said limiter arrangement is adapted for setting a predefined upper limit of the amount of nicotine administrated of a single administration.

According to an advantageous embodiment of the invention, said limiter arrangement is adapted for setting a predefined upper limit of the amount of nicotine administrated over several administrations, such as over a time interval of at least 1 hour, such as at least 2 hours, such as at least 6 hours, such as at least 12 hours, such as at least 24 hours.

According to an advantageous embodiment of the invention, said oral delivery system comprises one or more taste masking agents, such as sweetener and/or flavor, in a component.

As an example, said one or more taste masking agents may be a part of said first component, said second component, or a third component.

According to an embodiment of the invention, output opening(s) may comprise a nozzle for facilitating an increased output velocity of the component(s) administrated through said output opening(s).

### Figures

The invention will be described in the following with reference to the figures in which
figure 1A illustrates an oral delivery system according to an embodiment,
figure 1B illustrates an oral delivery system according to an embodiment,
figure 2A illustrates a part of an oral delivery system according to an embodiment,
figure 2B illustrates a part of an oral delivery system according to an embodiment,
figure 3 illustrates an oral delivery system according to an embodiment,
figure 4A illustrates a part of an oral delivery system according to an embodiment,
figure 4B illustrates a part of an oral delivery system according to an embodiment,
figure 4C illustrates a part of an oral delivery system according to an embodiment,
figure 5 illustrates a part of an oral delivery system according to an embodiment,
figure 6 illustrates an oral delivery system according to an embodiment,
figure 7A-E illustrate the timing of dispensing of components from an oral delivery system according to different embodiments,
figure 8 illustrates an oral delivery system according to an embodiment,
figure 9A illustrates an oral delivery system according to an embodiment,
figure 9B illustrates an oral delivery system according to an embodiment,
figure 10A illustrates an oral delivery system according to an embodiment,
figure 10B illustrates an oral delivery system according to an embodiment,
figure 11A illustrates an oral delivery system according to an embodiment, and
figure 11B illustrates an oral delivery system according to an embodiment.

### Detailed description of the invention

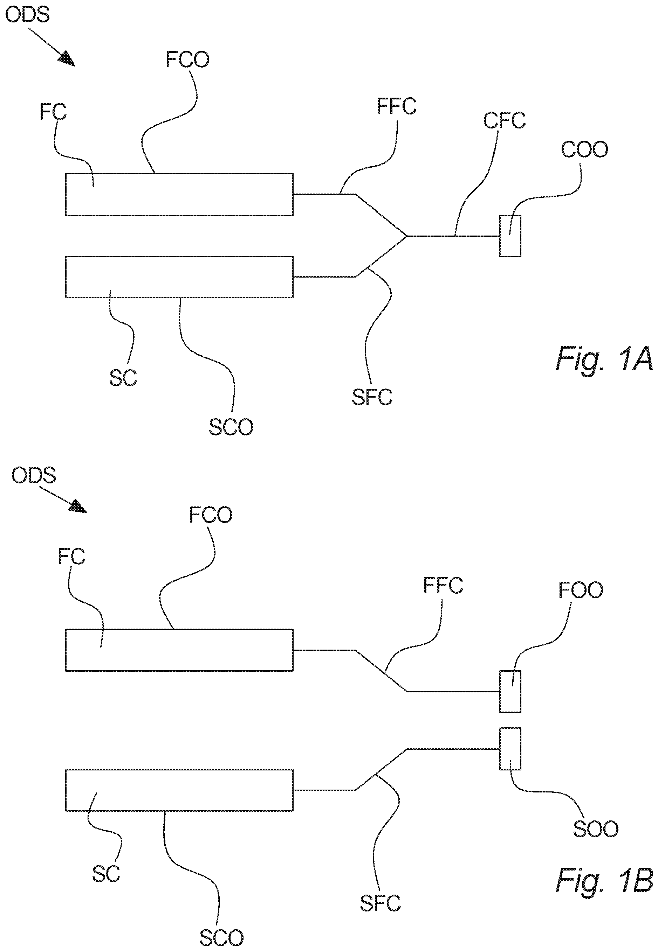

Fig.1A illustrates an embodiment of an oral delivery system ODS of the invention. The illustrated device comprises a first compartment FCO and a second compartment SCO having a first fluid conduit FFC and a second fluid conduit SFC, respectively. The first compartment FCO comprises a first component FC and the second compartment SCO comprises a second component SC.

The fluid conduits FFC and SFC merge fluid from the first and second compartments into a common fluid conduit CFC and may output fluid through a common output opening COO.

The common output opening COO may e.g. comprise a nozzle.

Fig. 1B illustrates an alternative oral delivery system ODS.

The illustrated device comprises a first compartment FCO and a second compartment SCO having a first fluid conduit FFC and a second fluid conduit SFC, respectively. The first compartment FCO comprises a first component FC and the second compartment SCO comprises a second component SC.

The fluid conduits FFC and SFC may output fluid from the first and second compartments into first fluid conduit FFC and second fluid conduit SFC to first output opening FOO and second output opening SOO, respectively.

The output openings may e.g. comprise a nozzle each.

The oral delivery system may also comprise further compartments, if it is desired to keep different components separated until the components are outputted from the compartment, such as a container.

An example of such configuration may e.g. be a three container version, where two containers comprise two separate components to be used together as a film forming agent and a third container comprising nicotine. Here, it should be understood that said first and second components together constitute said film forming agent, by means of at least the natural unbranched polysaccharides in said first component and the multivalent cations in said second component. The first and second components may comprise further compounds, additives, and/or auxiliary substances, which may or may not, according to different embodiments, form part of said film forming agent.

The containers mays typically be kept under pressure by means of conventional spray techniques with the use of a pressurized component or the components may e.g. simply be comprised in the containers ready for outputting via suitable mechanical or electromechanical pressure generation by means of external pressure inputted to the containers by an external pressure aggregate(s) (not shown).

The above described oral delivery systems may have different added or alternative features described in the following for synchronous outputting of two components.

In particular it should be noted that the below features and structures may advantageously all be applied in a three-compartment device, a four compartment device, or a multi compartment device having more than four compartments, if need may be.

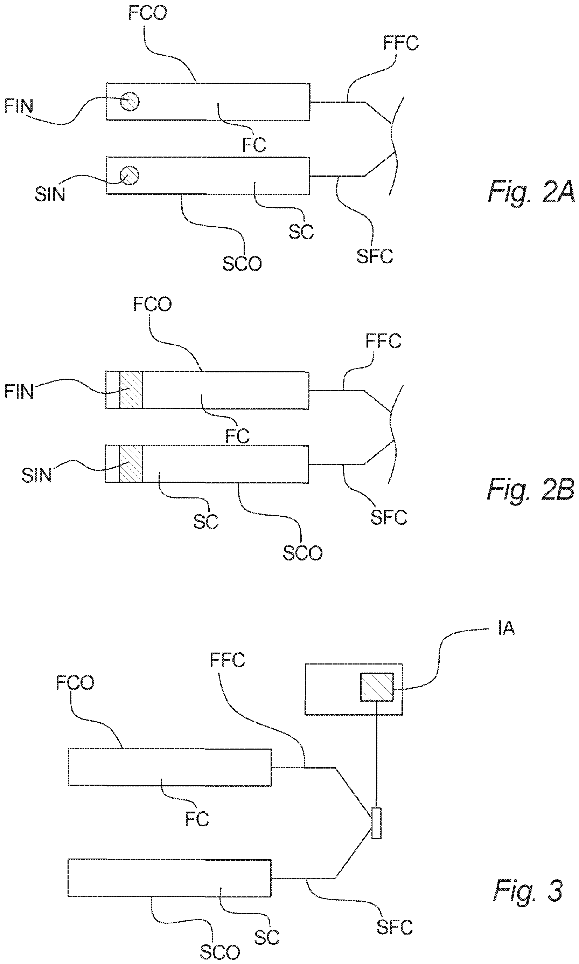

Fig. 2A and 2B illustrate two different variations of oral delivery systems ODS including indicators.

Both systems comprise a first and a second compartment FCO and SCO comprising a first component FC and a second component SC, respectively.

The containers may output first component FC and a second component SC through first and second output conduit FOC, SOC to first output opening FOO and second output opening SOO, respectively.

The first illustrated embodiment in fig. 2A comprises two active indicators FIN and SIN, by means of which a user may see whether the individual container still comprises a suitable amount of the components in the compartments FCO and SCO. The active indicators may e.g. communicate with an active measuring device by means of suitable equipment (not shown).

The second illustrated embodiment in fig. 2B, comprises two passive indicators FIN and SIN, by means of which a user may see whether the individual container still comprises a suitable amount of the components in the compartments FCO and SCO. The passive indicators may e.g. be formed by a translucent part of the compartment, through which a user may visually verify whether the desired components are still present.

Fig. 3 illustrates an embodiment of an oral delivery system ODS of the invention comprising an indicator. The illustrated device comprises a first compartment FCO and a second compartment SCO having a first fluid conduit FFC and a second fluid conduit SFC, respectively. The first compartment FCO comprises a first component FC and the second compartment SCO comprises a second component SC.

The first fluid conduit FFC and a second fluid conduit SFC leads the first component FC and the second components SC to respective opening(s) (not shown). The opening(s) may be configured for output of mixed components or individual components as illustrated in principle in fig. 1A and fig. 1B.

The illustrated delivery system furthermore comprises an indicator arrangement IA. The indicator arrangement IA is established as an indicator, which measures on the output of the compartments rather than the content of the compartments as in fig. 2A and 2B. This is in particular advantageous if the compartments are exchangeable and if it is desirable to keep the costs related manufacture to the individual containers with content as low as possible.

By the above internal or external measuring, it may e.g. be possible to measure on whether one or all of the compartments should be replaced with a new one.

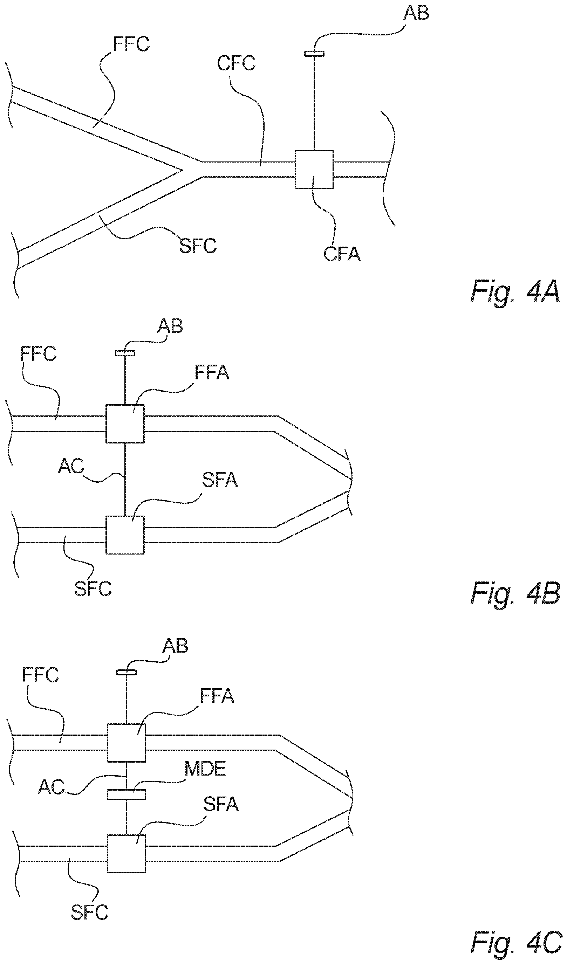

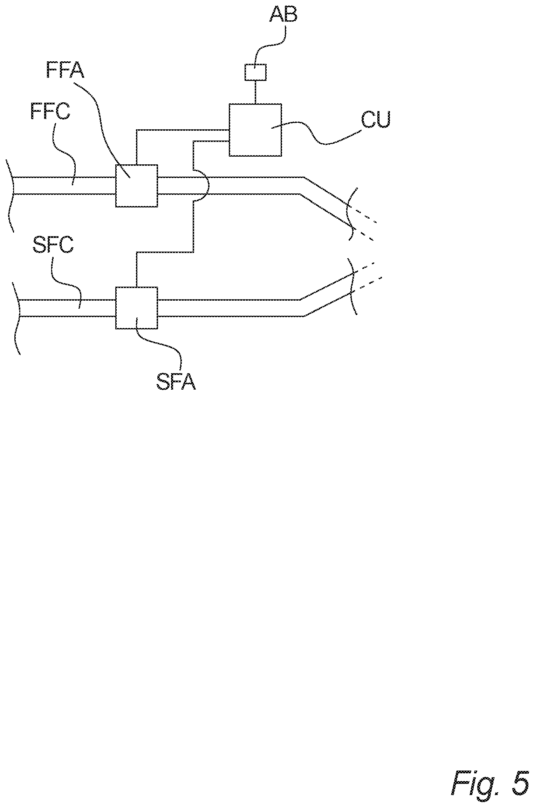

Figs. 4A-C and fig.5 are different examples directed to the output system availing the user to output components for the containers of the oral delivery system in the desired dose.

The output systems may e.g. be applied in the previous examples of fig. 1-3.

It should be noted that the illustrated embodiments are primarily directed a system where the coupled compartments are either pressurized, e.g. as aerosol containers or where the pressure in the containers - or sub-pressure is established externally to the containers when the output system is activated. The first type may e.g. be a typical aerosol can, suitable for the purpose, and the latter type may e.g. include a hand/finger pump, applied for the purpose of an output pressure only, when activated.

Fig. 4A illustrates a further oral delivery system including an output arrangement provided for administering the dose of components output from the system.

The below illustrated output arrangement may in principle be applied in any the above described delivery systems. The below examples are shown with devices comprising two compartments, but three or further numbers of compartments may be applied.

The output system includes the outer ends of a first fluid conduit FFC and a first fluid conduit SFC from respective compartments (not shown) merged together in one common fluid conduit CFC (not shown) to lead fluid to a common output opening COO (not shown).

The output system comprises a common flow actuator CFA which is operated mechanically by means of an activator button AB.

The activator button AB is mechanically coupled to the common flow actuator CFA, so as to enable a user to control the doses output from the at least two compartments (not shown) of the oral delivery system ODS via the output opening (not shown).

The illustrated activator button AB and the associated output system may be an all-mechanical arrangement.

Fig. 4B illustrates a two compartment version of the above delivery system.

The output system includes the outer ends of a first fluid conduit FFC and a first fluid conduit SFC from respective compartments (not shown) coupled to either a common output opening COO (not shown) as illustrated in fig. 1A or individual output openings (not shown) as illustrated in fig. 1B via respective first and second flow actuators FFA, SFA. The first and second flow actuators FFA, SFA are operated by means of an actuator button AB.

The illustrated embodiment comprises an actuator connection AC between the actuators arranged to transfer mechanical forces to both actuators FA, SA. The actuator connection may of course also be a connection from the actuator button directly to the individual flow actuators FFA, SFA. The important feature is that the actuators may be operated by a user by means of the actuator button to provide a synchronous from the first and second compartment and the output from the first and second compartment are mixed either before leaving the output arrangement or is mixed externally, e.g. in the oral cavity, where a user is targeting the output.

Fig. 4C illustrates a two compartment version according to an embodiment of the invention.

The output system includes the outer ends of a first fluid conduit FFC and a second fluid conduit SFC from respective compartments (not shown) coupled to either a common output opening COO (not shown) as illustrated in fig. 1A or individual output openings (not shown) as illustrated in fig. 1B via respective first and second flow actuators FFA, SFA. The first and second actuators FFA, SFA are operated by means of an actuator button AB.

The illustrated embodiment comprises an actuator connection AC between the actuators arranged to transfer mechanical forces to both flow actuators FFA, SFA via a mechanical delay element MDE. The mechanical delay element is a primitive mechanical element arranged to transfer mechanical movement between the actuators with a certain predefined delay. In this way, it may be possible to provide a synchronous outputting from the feeding compartments, while e.g. providing a delay in the opening of the second fluid conduit relative to the first fluid conduit.

Such a delay may e.g. be advantageous insofar priming of the mucosa prior to the dosing of nicotine is desired.

Fig. 5 illustrates a further advantageous embodiment of the invention.
The below illustrated output arrangement may in principle be applied in any the above described delivery systems. The below examples are shown with devices comprising two compartments, but three or further numbers of compartments may be applied.

The output system includes the outer ends of a first fluid conduit FFC and a second fluid conduit SFC of respective compartments (not shown) optionally merged together in one common fluid conduit CFC (not shown) to lead fluid to a common output opening COO (not shown).

The illustrated oral delivery system may be applied in any of the previous embodiments of fig. 1-3.

The illustrated output system includes the outer ends of a first fluid conduit FFC and a first fluid conduit SFC from respective compartments (not shown) coupled to either a common output opening COO (not shown) as illustrated in fig. 1A or individual output openings (not shown) as illustrated in fig. 1B via respective first and second flow actuators FFA, SFA. The first and second flow actuators FFA, SFA are electromechanical and they are activated by a user by means of an actuator button AB via a control unit CU.

The control unit and the electromechanical actuators FFA and SFA are electrically powered, preferable by means of a battery.

The electromechanical version of the delivery system has several benefits over the mechanical versions insofar a more complicated controlling of the administration is desired. By means of individually controlled actuators, it may be possible to obtain a very customized and precise delivery to the mucosa, due to the fact that the actuators may be individually controlled to provide the exact mixture at the right time.

Due to the higher manufacturing costs of the more complex electromechanical delivery system, the compartments may preferably be exchangeable or refillable.

Evidently, the application of exchangeable or refillable compartments may also be advantageous in the previous simple mechanical system.

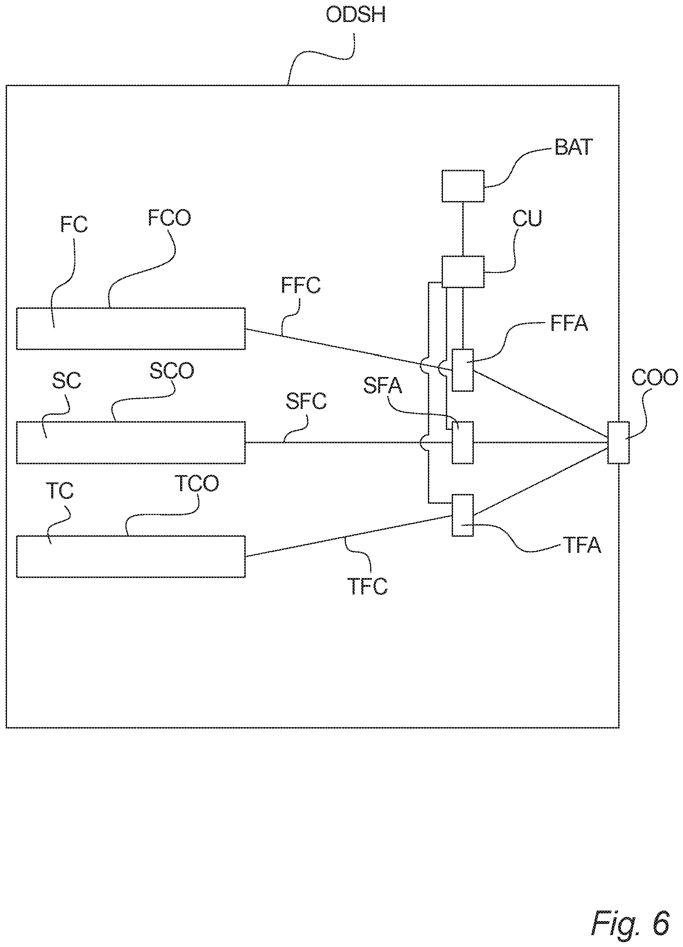

Fig. 6 illustrates a device according to an example embodiment, the device comprising three compartments FCO, SCO, TCO each comprising a component FC, SC, TC. The compartments are each connected to a common output opening COO via separate fluid conduits FFC, SFC, TFC. On each of the fluid conduits a fluid actuator FFA, SFA, TFA is positioned so as to regulate the flow of said components from their respective compartments, through said fluid conduits, to the common output opening COO. Here the fluid actuators FFA, SFA, TFA are electromechanical and separately controlled by a control unit CU via separate electrical connections. The control unit CU is powered by a battery BAT. As can be seen, the device is enclosed in a oral delivery system housing ODSH, and only the common output opening is connected to the outside of said housing so as to dispense components from their compartments inside said housing to the outside of the housing, e.g. to the oral cavity.

In connection with the above it should be understood that the battery may power other parts of the oral delivery system, such as e.g. the fluid actuators, and may preferably power all parts of the oral delivery system in need of powering.

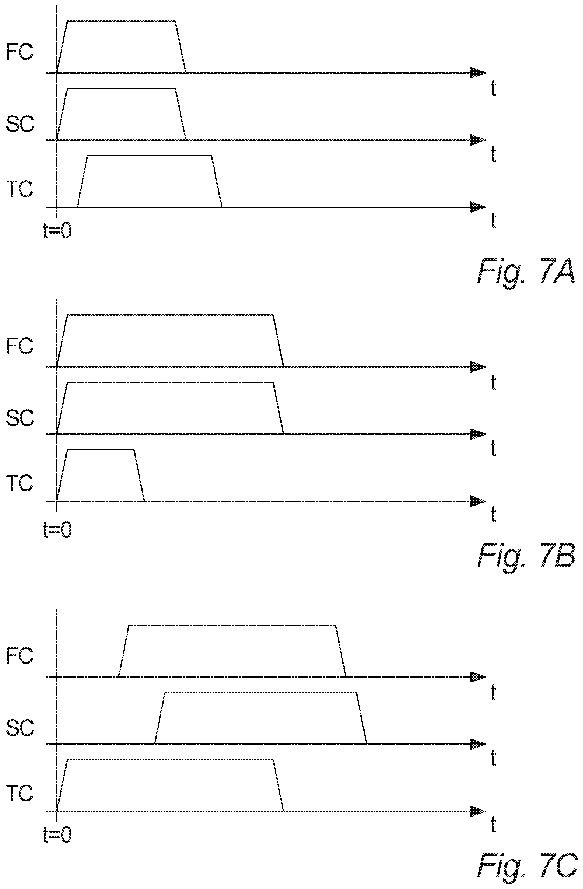

Fig. 7A-E illustrate different advantageous synchronous administrations timings, which may be applied and established by means of the above electromechanical system.

In all the illustrated examples, FC, SC and TC illustrate the actuator timing in relation to the dispensing of the first component, the second component and the third component, respectively.

The illustrated timings are all related to a three compartment version of the oral delivery system ODS, e.g. as illustrated in the embodiment of fig. 6. The timings may evidently be modified to fit into two, four or further compartment delivery systems.

Fig. 7A illustrates, by an example, that two components are dispensed simultaneous and the after a delay in timing, the third component may be dispensed. Such a solution may e.g. be advantageous if the first and the second component comprises film forming agents and the third component comprises nicotine, given the fact that nicotine is very expensive and that the uptake of nicotine through mucosa should be optimized.

Fig. 7B illustrates another example of dispensing the first, second, and third components FC, SC, TC. The first and second components form the film forming agent. The third component comprises nicotine. As illustrated, the dispensing of the first and second components continues after termination of dispensing of the third component. Thereby, the first part of the bioadhesive film formed from the first and second components will contain nicotine from the third component, while a nicotine-free film is formed on top of the first part of the bioadhesive film. Thereby, the dispensed nicotine is protected by the additional nicotine-free layer, and the use of the very expensive nicotine is minimized, so as to reduce waste of nicotine in the outer (latest formed) part of the film from which nicotine transfer to the oral mucosa may be significantly less effective.

Fig. 7C illustrates yet another example of dispensing the three components FC, SC, TC in an advantageous way. Here, the first and second components constitute film forming agent, while the third component comprises nicotine. It may be advantageous to start dispensing only with the nicotine-containing third component, thereby building up a relatively high concentration of nicotine on the oral mucosa. Then, dispensing of the first component is started. The first component may in some embodiments have a relatively high viscosity, at least compared to the second component, and may therefore be substantially stationary on the oral mucosa in the time interval until the initiation of the dispensing of the second component, which triggers the cross-linking film forming reaction. Again, dispensing of nicotine is avoided in the outer part of the film. As illustrated, it may in some cases be advantageous to continue dispensing the second component after dispensing of the other components is terminated. For example, in some cases, it may give rise to an advantageous termination of the bioadhesive film, by securing that the outer layer is saturated with multivalent cations. Also, in embodiments where the components are dispensed from a common output opening, e.g. a common nozzle, it may be advantageous to flush the shared part of the dispensing system with clean second component so as to avoid or at least reduce residue mixture of the first and second components, which may clog the output opening by forming a film.
Alternatively, the first and second components may be synchronized to be dispensed at the same time to speed up the film forming process.

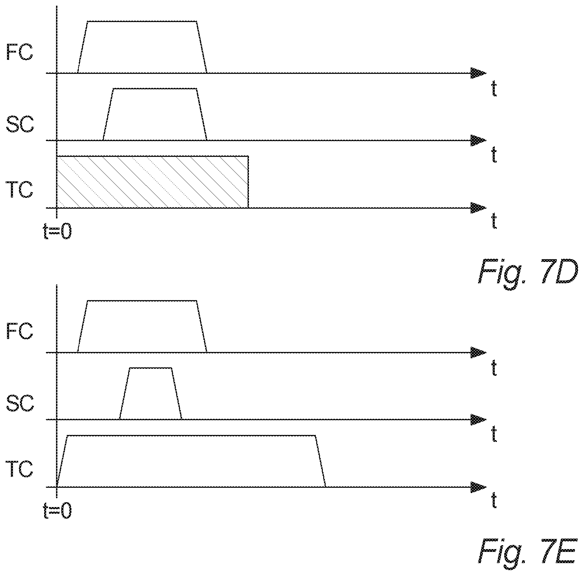

Fig. 7D illustrates another example of dispensing the three components FC, SC, TC. Here, the first and second components constitute film forming agent, while the third component comprises nicotine. The hatched area illustrates that the timing of the third component may be varied due to circumstances and objectives of the particular oral delivery system, e.g. as illustrated on fig. 7A-C, but should preferably fall substantially within at least that hatched area to achieve immobilization of the nicotine on the oral mucosa.

Fig. 7E illustrates another example of dispensing the three components FC, SC, TC. Here, the first and second components FC, SC constitute film forming agent, while the second component also comprises nicotine. The third component TC comprises one or more taste masking agents, such as sweetener and/or flavor. As illustrated, it may in some cases be advantageous to only dispense the nicotine-containing second component SC for a short while to avoid or reduce waste of the expensive nicotine.

Also, the taste masking agent-containing third component TC may be dispensed throughout the dispending of the first and second components FC, SC, and preferably also for a while after to eliminate or at least reduce displeasure from the taste of nicotine.

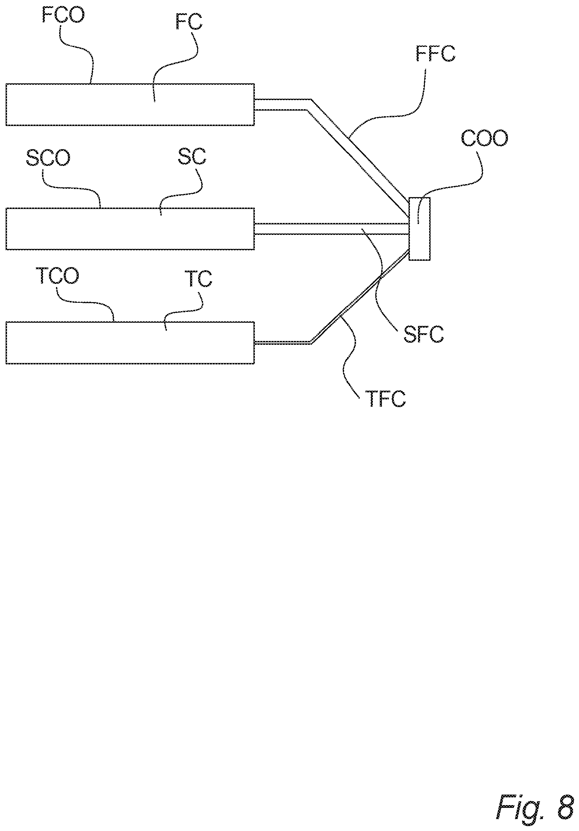

Fig. 8 illustrates another example embodiment, where three components FC, SC, TC in separate compartments FCO, SCO, TCO may be administered through separate fluid conduits FFC, SFC, TFC to a common output opening COO. Here one important detail is that each flow conduit may be adapted in size so as to be adapted to feed an amount of the particular component with a desired pre-defined velocity (volume per time). Specifically, in this embodiment, the third component may comprise nicotine whereas the first and second components together form the film forming agent. Due to the fact that nicotine may be a very expensive substance, the third fluid conduit TFC is here show having a reduced diameter, so as to be able to adjust the dispensed amount more precisely. In alternative embodiments, the first and second fluid conduits FFC, SFC may also be different sized flow conduits. For example, if a very concentrated solution of multivalent cations is used, a narrow fluid conduit SFC may be used to facilitate more accurate administration of this second component SC.

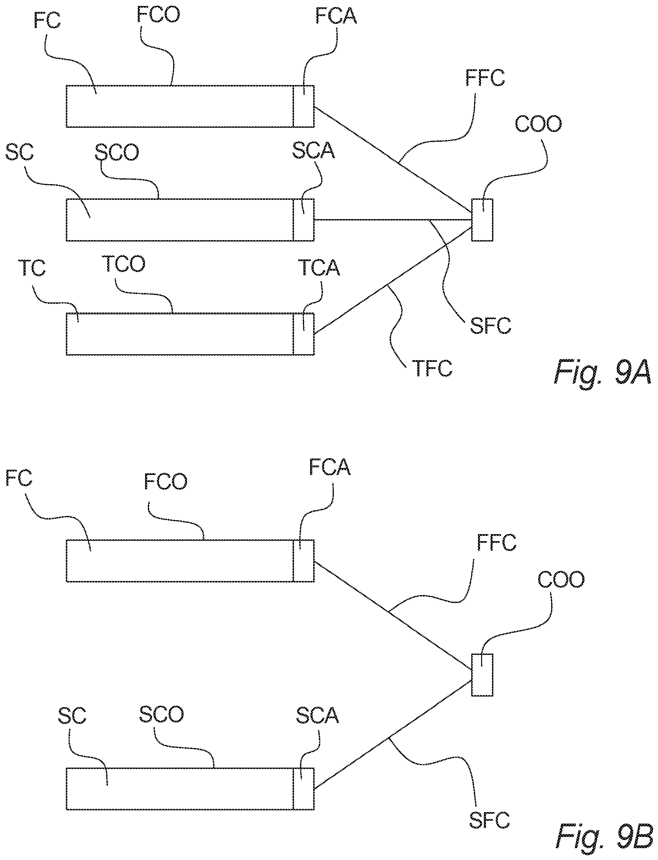

Fig. 9A and 9B illustrate devices according to further example embodiments.

On figure 9A three compartments FCO, SCO, TCO comprises first, second, and third components FC, SC, TC, respectively. Each of the compartments are connected via fluid conduits FFC, SFC, TFC to a common output opening COO, as illustrated in connection with previous figures. On fig. 9A, each of the compartments FCO, SFO, TFO comprises a connection arrangement FCA, SCA, TCA for connecting the compartment to the rest of the system. Thereby, it may be facilitated that each of the compartments are replaceable, either by the end user, or by a specialist. In an alternative embodiment, the compartments may be connected to a common connection arrangement thereby facilitating replaceability as a single cartridge.

In fig. 9B is illustrated an alternative to the oral delivery system of fig. 9A, the device of fig. 9B being a two-compartment device. Otherwise, the connection arrangements and the replaceability may be devised as in fig. 9A.

The above-described embodiments are in their preferred form portable.

In the above described nicotine oral spray versions of the delivery system the compartment may typically be more correctly referred to be containers. The containers should preferably be able to sustain pressure either permanently provided in the containers or e.g. provided only during the activation of the oral delivery systems output system.

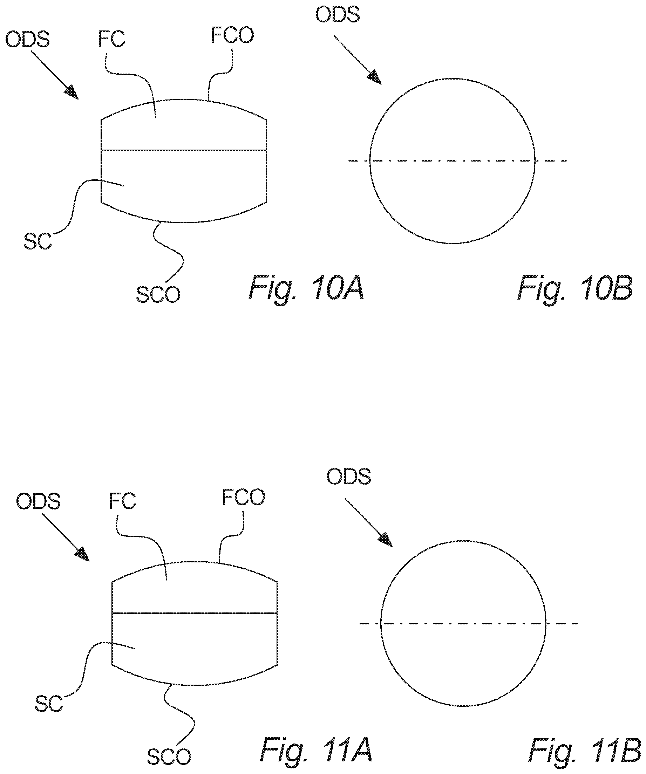

Fig. 10A and 10B illustrates an alternative oral delivery system ODS, where the oral delivery system ODS is a chewing gum. The chewing gum, being a multi-module chewing gum, is here shown as a two-layer chewing gum, but the modules may not necessarily be layers.The chewing gum may comprise additional modules. As shown the oral delivery system ODS is a chewing gum comprising a first compartment FCO shown as one layer and a second compartment SCO shown as one other layer connected to, but separate from the layer forming the first compartment. The first compartment FCO comprises the first component FC, while the second compartment comprises the second component SC. When the chewing gum is administered to the oral cavity, the first and second components FC, SC will start to dissolve when contacted with saliva in the oral cavity and/or when chewed, and mixing will occur in the mouth triggering the cross-linking reaction. The chewing gum comprises gum base in the first and/or second compartment. The chewing gum may advantageously comprise an active ingredient, such as nicotine, in the first and/or second component. The chewing gum may for example be made by compression.

Fig. 11A and 11B illustrates an alternative oral delivery system ODS, where the oral delivery system ODS is a lozenge. The lozenge, being a multimodule lozenge, is here shown as a two-layer lozenge, but the modules may not necessarily be layers.The lozenge may comprise additional modules. As shown the oral delivery system ODS is a lozenge comprising a first compartment FCO shown as one layer and a second compartment SCO shown as one other layer connected to, but separate from the layer forming the first compartment. The first compartment FCO comprises the first component FC, while the second compartment comprises the second component SC. When the lozenge is administered to the oral cavity, the first and second components FC, SC will start to dissolve when contacted with saliva in the oral cavity and mixing will occur in the mouth triggering the cross-linking reaction. The lozenge may advantageously comprise an active ingredient, such as nicotine, in the first and/or second component. The lozenge may for example be made by compression.

### List of figure references

- ODS.: Oral delivery system
- FCO.: First compartment
- SCO.: Second compartment
- TCO.: Third compartment
- FC.: First component
- SC.: Second component
- TC.: Third component
- FFC.: First fluid conduit
- SFC.: Second fluid conduit
- CFC.: Common fluid conduit
- TFC.: Third fluid conduit
- FOO.: First output opening
- SOO.: Second output opening
- COO.: Common output opening
- FIN.: First indicator
- SIN.: Second indicator
- FFA.: First flow actuator
- SFA.: Second flow actuator
- TFA.: Third flow actuator
- CFA.: Common flow actuator
- CU.: Control unit
- OSDH.: Oral delivery system housing
- BAT.: Battery
- AB.: Activator button
- FCA.: First connection arrangement
- SCA.: Second connection arrangement
- TCA.: Third connection arrangement
- IA.: Indicator arrangement
- MDE.: Mechanical delay element
- AC.: Actuator connection

## Claims

1. An oral delivery system (ODS) comprising a first compartment (FCO) and a second compartment (SCO),
said first compartment (FCO) comprising a first component (FC) comprising natural unbranched polysaccharide,
said second compartment (SCO) comprising a second component (SC) comprising multivalent cations,
wherein the oral delivery system (ODS) is adapted for administering the first and second components (FC, SC) in a synchronized manner to the oral cavity,
whereby a bioadhesive gel is formed from said natural unbranched polysaccharide and said multivalent cations in a cross-linking reaction,
wherein the first and/or second components (FC, SC) comprise(s) at least one active ingredient,
wherein said active ingredient comprises nicotine,
wherein said natural unbranched polysaccharides comprise alginate,
wherein said oral delivery system (ODS) is adapted for delivering a pre-defined amount of nicotine to the oral cavity, where said pre-defined amount is 0.5 - 4 mg.

2. An oral delivery system (ODS) according to claim 1, wherein said cross-linking reaction occurs at least partly in the oral cavity.

3. An oral delivery system (ODS) according to claim 1 or 2, wherein the oral delivery system (ODS) is an oral spray system.

4. An oral delivery system (ODS) according to any of claims 1-3, wherein said nicotine is selected from the group consisting of a nicotine salt, the free base form of nicotine, a nicotine derivative, such as a nicotine cation exchanger, such as nicotine polacrilex resin, a nicotine inclusion complex or nicotine in any non-covalent binding; nicotine bound to zeolites; nicotine bound to cellulose, such as microcrystalline, or starch microspheres, and mixtures thereof.

5. An oral delivery system (ODS) according to any of claims 1-3, wherein nicotine is present as a nicotine polacrilex resin.

6. An oral delivery system (ODS) according to any of the preceding claims, wherein said first component (FC) and second component (SC) are adapted to form a bio-adhesive gel which adheres at least partly to the oral mucosa.

7. An oral delivery system (ODS) according to any of the preceding claims, wherein said first component (FC) and/or second component (SC) comprise(s) a pH-controlling agent, such as a buffer.

8. An oral delivery system (ODS) according to any of the preceding claims, wherein said pre-defined amount between a series of at least 10 of administrations from said oral delivery system (ODS) holds a relative standard deviation (RSD) of at most 6%.

9. An oral delivery system (ODS) according to any of the preceding claims, wherein said first component (FC) is in the form of aqueous solution or an aqueous suspension.

10. An oral delivery system (ODS) according to any of the preceding claims, wherein said multivalent cations comprise metal cations, and/or wherein said multivalent cations are selected from the group of divalent cations.

11. An oral delivery system (ODS) according to any of the preceding claims, wherein said multivalent cations are in the form of aqueous solution.

12. An oral delivery system (ODS) according to any of the preceding claims, wherein said first component (FC) and/or second component (SC) comprise(s) a gelling time controlling substance, wherein said gelling time controlling substance comprises a gelling delaying substance, wherein said gelling delaying substance comprises a polyol, such as sorbitol.

13. An oral delivery system (ODS) according to any of any of the preceding claims, wherein said first component (FC) and/or second component (SC) comprise(s) a gelling time controlling substance, wherein said gelling time controlling substance comprises a gelling accelerating substance, wherein said gelling accelerating substance comprises a pH-controlling substance, such as a buffer and/or an acid.

14. An oral delivery system (ODS) according to any of the preceding claims, wherein said oral delivery system (ODS) is adapted for administration of said first component (FC) and second component (SC) substantially at the same time.

## Patentansprüche

1. System zur oralen Verabreichung (ODS), das eine erste Kammer (FCO) und eine zweite Kammer (SCO) umfasst,
wobei die erste Kammer (FCO) einen ersten Bestandteil (FC) umfasst, der natürliches unverzweigtes Polysaccharid umfasst,
wobei die zweite Kammer (SCO) einen zweiten Bestandteil (SC) umfasst, der mehrwertige Kationen umfasst,
wobei das System zur oralen Verabreichung (ODS) dafür eingerichtet ist, den ersten und zweiten Bestandteil (FC, SC) in synchronisierter Weise an die Mundhöhle zu verabreichen,
wobei aus dem natürlichen unverzweigten Polysaccharid und den mehrwertigen Kationen in einer Vernetzungsreaktion ein bioadhäsives Gel gebildet wird,
wobei der erste und/oder zweite Bestandteil (FC, SC) wenigstens einen Wirkstoff umfasst/umfassen,
wobei der Wirkstoff Nicotin umfasst,
wobei die natürlichen unverzweigten Polysaccharide Alginat umfassen,
wobei das System zur oralen Verabreichung (ODS) dafür eingerichtet ist, eine vorab festgelegte Menge an Nicotin an die Mundhöhle zu verabreichen, wobei die vorab festgelegte Menge 0,5 bis 4 mg beträgt.

2. System zur oralen Verabreichung (ODS) nach Anspruch 1, wobei die Vernetzungsreaktion wenigstens teilweise in der Mundhöhle stattfindet.

3. System zur oralen Verabreichung (ODS) nach Anspruch 1 oder 2, wobei das System zur oralen Verabreichung (ODS) ein Mundspraysystem ist.

4. System zur oralen Verabreichung (ODS) nach einem der Ansprüche 1 bis 3, wobei das Nicotin aus der Gruppe ausgewählt ist, die aus Folgendem besteht: einem Nicotinsalz, der freie-Base-Form von Nicotin, einem Nicotinderivat, wie einem Nicotin-Kationenaustauscher, wie Nicotin-Polacrilex-Harz, einem Nicotin-Einschlusskomplex oder Nicotin in einer jeglichen nicht-kovalenten Bindung; an Zeolithe gebundenem Nicotin; an Cellulose, wie mikrokristalline Cellulose oder Stärke-Mikrosphären, gebundenem Nicotin, und Mischungen von diesen.

5. System zur oralen Verabreichung (ODS) nach einem der Ansprüche 1 bis 3, wobei Nicotin als ein Nicotin-Polacrilex-Harz vorliegt.

6. System zur oralen Verabreichung (ODS) nach einem der vorangegangenen Ansprüche, wobei der erste Bestandteil (FC) und der zweite Bestandteil (SC) dafür eingerichtet sind, ein bioadhäsives Gel zu bilden, das wenigstens teilweise an der Mundschleimhaut haftet.

7. System zur oralen Verabreichung (ODS) nach einem der vorangegangenen Ansprüche, wobei der erste Bestandteil (FC) und/oder der zweite Bestandteil (SC) ein pH-regulierendes Mittel, wie einen Puffer, umfasst/umfassen.

8. System zur oralen Verabreichung (ODS) nach einem der vorangegangenen Ansprüche, wobei in einer Reihe von wenigstens 10 Verabreichungen aus dem System zur oralen Verabreichung (ODS) die vorab festgelegte Menge eine relative Standardabweichung (RSD) von höchstens 6% einhält.

9. System zur oralen Verabreichung (ODS) nach einem der vorangegangenen Ansprüche, wobei der erste Bestandteil (FC) in Form einer wässrigen Lösung oder einer wässrigen Suspension vorliegt.

10. System zur oralen Verabreichung (ODS) nach einem der vorangegangenen Ansprüche, wobei die mehrwertigen Kationen Metallkationen umfassen und/oder wobei die mehrwertigen Kationen aus der Gruppe von zweiwertigen Kationen ausgewählt sind.

11. System zur oralen Verabreichung (ODS) nach einem der vorangegangenen Ansprüche, wobei die mehrwertigen Kationen in Form einer wässrigen Lösung vorliegen.

12. System zur oralen Verabreichung (ODS) nach einem der vorangegangenen Ansprüche, wobei der erste Bestandteil (FC) und/oder der zweite Bestandteil (SC) eine die Gelbildungszeit regulierende Substanz umfasst/umfassen, wobei die die Gelbildungszeit regulierende Substanz eine die Gelbildung verzögernde Substanz umfasst, wobei die die Gelbildung verzögernde Substanz ein Polyol, wie Sorbit, umfasst.

13. System zur oralen Verabreichung (ODS) nach einem der vorangegangenen Ansprüche, wobei der erste Bestandteil (FC) und/oder der zweite Bestandteil (SC) eine die Gelbildungszeit regulierende Substanz umfasst/umfassen, wobei die die Gelbildungszeit regulierende Substanz eine die Gelbildung beschleunigende Substanz umfasst, wobei die die Gelbildung beschleunigende Substanz eine pH-regulierende Substanz, wie einen Puffer und/oder eine Säure, umfasst.

14. System zur oralen Verabreichung (ODS) nach einem der vorangegangenen Ansprüche, wobei das System zur oralen Verabreichung (ODS) für eine im Wesentlichen gleichzeitige Verabreichung des ersten Bestandteils (FC) und des zweiten Bestandteils (SC) eingerichtet ist.

## Revendications

1. Système d'administration orale (ODS) comprenant un premier compartiment (FCO) et un deuxième compartiment (SCO),
ledit premier compartiment (FCO) comprenant un premier composant (FC) comprenant un polysaccharide naturel non ramifié,
ledit deuxième compartiment (SCO) comprenant un deuxième composant (SC) comprenant des cations multivalents,
le système d'administration orale (ODS) étant adapté pour administrer les premier et deuxième composants (FC, SC) d'une manière synchronisée à la cavité buccale,
dans lequel un gel bioadhésif est formé à partir dudit polysaccharide naturel non ramifié et desdits cations multivalents dans une réaction de réticulation,
dans lequel le(s) premier et/ou deuxième composants (FC, SC) comprend/comprennent au moins une substance active, dans lequel ladite substance active comprend la nicotine,
dans lequel lesdits polysaccharides naturels non ramifiés comprennent un alginate, ledit système d'administration orale (ODS) étant adapté pour administrer une quantité prédéfinie de nicotine à la cavité buccale, où ladite quantité prédéfinie est 0,5 à 4 mg.

2. Système d'administration orale (ODS) selon la revendication 1, dans lequel ladite réaction de réticulation se produit au moins partiellement dans la cavité buccale.

3. Système d'administration orale (ODS) selon la revendication 1 ou 2, le système d'administration orale (ODS) étant un système de pulvérisation orale.

4. Système d'administration orale (ODS) selon l'une quelconque des revendications 1 à 3, dans lequel, ladite nicotine est choisie dans le groupe constitué d'un sel de nicotine, la forme de base libre de nicotine, un dérivé de nicotine, tel qu'un échangeur de cations de nicotine, tel que la résine de nicotine polacrilex, un complexe d'inclusion de nicotine ou la nicotine dans une liaison non covalente quelconque ; la nicotine liée à des zéolites ; la nicotine liée à une cellulose, telle que la microcristalline, ou des microsphères d'amidon, et des mélanges de celles-ci.

5. Système d'administration orale (ODS) selon l'une quelconque des revendications 1 à 3, dans lequel la nicotine est présente sous la forme d'une résine de nicotine polacrilex.

6. Système d'administration orale (ODS) selon l'une quelconque des revendications précédentes, dans lequel lesdits premier composant (FC) et deuxième composant (SC) sont adaptés pour former un gel bioadhésif qui adhère au moins partiellement à la muqueuse buccale.

7. Système d'administration orale (ODS) selon l'une quelconque des revendications précédentes, dans lequel ledit/lesdits premier composant (FC) et/ou deuxième composant (SC) comprend/comprennent un agent de régulation du pH, tel qu'un tampon.

8. Système d'administration orale (ODS) selon l'une quelconque des revendications précédentes, dans lequel ladite quantité prédéfinie entre une série d'au moins 10 administrations à partir dudit système d'administration orale (ODS) maintient un écart type relatif (RSD) d'au plus 6 %.

9. Système d'administration orale (ODS) selon l'une quelconque des revendications précédentes, dans lequel ledit premier composant (FC) est sous la forme d'une solution aqueuse ou d'une suspension aqueuse.

10. Système d'administration orale (ODS) selon l'une quelconque des revendications précédentes, dans lequel lesdits cations multivalents comprennent des cations métalliques, et/ou dans lequel lesdits cations multivalents sont choisis dans le groupe de cations divalents.

11. Système d'administration orale (ODS) selon l'une quelconque des revendications précédentes, dans lequel lesdits cations multivalents sont sous forme de solution aqueuse.

12. Système d'administration orale (ODS) selon l'une quelconque des revendications précédentes, dans lequel ledit/lesdits premier composant (FC) et/ou deuxième composant (SC) comprend/comprennent une substance de régulation du temps de gélification, dans lequel ladite substance de régulation du temps de gélification comprend une substance retardant la gélification, dans lequel ladite substance retardant la gélification comprend un polyol, tel que le sorbitol.

13. Système d'administration orale (ODS) selon l'une quelconque des revendications précédentes, dans lequel ledit/lesdits premier composant (FC) et/ou deuxième composant (SC) comprend/comprennent une substance de régulation du temps de gélification, dans lequel ladite substance de régulation du temps de gélification comprend une substance accélérant la gélification, dans lequel ladite substance accélérant la gélification comprend une substance de régulation du pH, telle qu'un tampon et/ou un acide.

14. Système d'administration orale (ODS) selon l'une quelconque des revendications précédentes, ledit système d'administration orale (ODS) étant adapté pour l'administration desdits premier composant (FC) et deuxième composant (SC) sensiblement en même temps.
